Europäisches Patentamt

European Patent Office

Office européen des brevets

(11) Publication number: **0 174 740**
**B1**

# EUROPEAN PATENT SPECIFICATION

(45) Date of publication of patent specification: **26.07.89**

(51) Int. Cl.⁴: **A 46 B 15/00**, A 46 B 7/04,
A 46 B 17/08, A 61 N 1/26

(21) Application number: **85305725.5**

(22) Date of filing: **13.08.85**

(54) Electric tooth-brush.

(30) Priority: 14.08.84 JP 169551/84
16.09.84 JP 193504/84
05.05.85 JP 95745/85

(43) Date of publication of application:
**19.03.86 Bulletin 86/12**

(45) Publication of the grant of the patent:
**26.07.89 Bulletin 89/30**

(84) Designated Contracting States:
**CH DE FR GB IT LI**

(56) References cited:
**EP-A-0 016 564
FR-A- 750 214
FR-A-1 538 649
FR-A-1 558 852
FR-A-2 218 860
FR-E- 95 197
GB-A- 202 424
GB-A- 390 985
GB-A- 956 126
US-A-1 887 913
US-A-2 219 753**

(73) Proprietor: **Hukuba, Hiroshi
No. 914-1, Nazukari
Nagareyama-shi Chiba-ken (JP)**

(72) Inventor: **Hukuba, Hiroshi
No. 914-1, Nazukari
Nagareyama-shi Chiba-ken (JP)**

(74) Representative: **Newstead, Michael John et al
Page & Co. Temple Gate House Temple Gate
Bristol BS1 6PL (GB)**

(56) References cited:
**US-A-2 722 703
US-A-3 271 805
US-A-3 488 788
US-A-3 667 454
US-A-4 227 276**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

This invention relates to an electric tooth-brush.

A known electric tooth-brush has a handle comprising a head portion studded with bristles and a grip portion removably coupled to the head portion. In the head portion, a head conductor member is embedded having one end exposed to the bristles and the other end exposed to the end of the head portion opposite the brush threads. The grip portion accommodates a battery and carries on its top surface a grip conductor member connected to the battery. When the grip portion is coupled to the head portion, the head conductor member is electrically connected to the battery. In use, the battery, the grip conductor member, a user's body and the head conductor member constitute a closed circuit which, when a tooth paste containing fluoride is used, causes electrical permeation of fluorine ions into the teeth, thereby ensuring protection and prevention against tooth decay and pyorrhea.

Generally, after repetitious use of a tooth-brush, its bristles will be worn and torn and the tooth-brush, now being of no use, must be disposed of. The usable term normally amounts to about one month.

When, in the known tooth-brush described above, wear and tear of the bristles occurs, the head portion is disposed of and replaced with a new one but the grip portion, not being subject to replacement, is put into further use. This leads to cost reduction as compared with the cost involved in disposal of the entire toothbrush. However, the head conductor member which is embedded in the head portion is inseparable therefrom and therefore, upon disposal of the head portion, the head conductor member is simultaneously disposed of even if it is still usable, resulting in a disadvantage of insufficient cost reduction. Furthermore, the head portion with the head conductor member embedded is complicated in structure.

According to the present invention, there is provided an electric tooth-brush comprising a grip portion which can be gripped manually, an elongate head portion connected to and separable from said grip portion and studded with bristles, a head conductor member, a grip conductor member which covers a surface of said grip portion and an electric power source connected to said head conductor member and said grip conductor member, characterised in that:-

said head conductor member is an elongate head conductor member which projects lengthwise from the end of said grip portion adjacent said head portion, said head portion has an elongate lengthwise extending receiving space in it, said head conductor member being carried by said grip portion and extending slidably into said receiving space and terminating at a location within said receiving space, which location is spaced from said bristles, and at least a part of the length of said head conductor member being covered by the part of the head portion that defines said receiving space; and

fluid communication passage means extends from said bristles to said receiving space so that fluid can flow from said bristles to said head conductor member in said receiving space.

The present invention will now be described by way of example with reference to the accompanying drawings, in which:-

Fig. 1 is a plan view showing a grip portion and a head portion, illustrated as separated from each other, of an electric tooth-brush according to a first embodiment of the invention;

Fig. 2 is an underneath view of the Fig. 1 embodiment after assembly;

Fig. 3 is a longitudinal sectional view taken along line 3-3 in Fig. 2;

Fig. 4 is a side view of the Fig. 1 embodiment;

Fig. 5 is a plan view showing a grip portion and a head portion, illustrated as separated from each other, of an electric tooth-brush according to a second embodiment of the invention;

Fig. 6 is an underneath view of the Fig. 5 embodiment after assembly;

Fig. 7 is a side view of the Fig. 5 embodiment;

Fig. 8 is an enlarged cross sectional view taken along line 8-8 in Fig. 7;

Fig. 9 is a plan view of an electric tooth-brush according to a third embodiment of the invention;

Fig. 10 is an underneath view of the Fig. 9 embodiment;

Fig. 11 is a side view of the Fig. 9 embodiment; and

Fig. 12 is an enlarged cross sectional view taken along line 12-12 in Fig. 11.

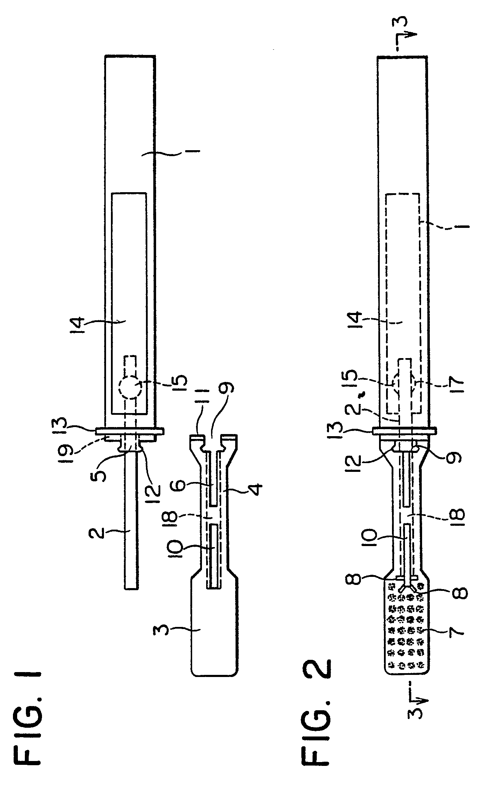

Referring to Figs. 1 to 4, there is shown an electric tooth-brush according to a first embodiment of the invention. The electric tooth-brush has a handle comprising a grip portion 1 and a head portion 3 removably coupled to the grip portion 1.

The grip portion 1 has, on its outer periphery near its end adjacent the head portion 3, an annular, water blocking collar 13. A boss 5 having projections 12 on opposite sides of its tip, extends from the end surface of the grip portion 1 nearer the head portion 3. In this end surface, there are grooves 19 each of which extends vertically in Fig. 1 and has a depth reaching the collar 13. A conductor bar 2 extends outwardly of the boss 5 and has a rear part which is inserted and fixed in the grip portion 1. The grip portion 1 is formed, near the rear end of the conductor bar 2, with a hole 17 which extends from an upper surface of the conductor bar 2 to a top surface of the grip portion 1. Within this hole 17, a spring 16 of a good electrically conductive material and a battery 15 are stacked sequentially from the conductor bar 2. The battery 15 has an upper positive pole and is held in the hole 17 with its upper surface engaged with a metal cover plate 14 carried or mounted on the grip portion 1.

The head portion 3 has, at its end adjacent the grip portion 1, a fork of two arms 4 between which there is formed a recess 9 substantially resembling the boss 5 for snug reception of the projections 12 and boss 5 and a slit 6 extending from the recess 9 in the axial direction in an elongate shank part of the head portion which extends away from the grip portion. Each of the arms 4 has a projection 11 which is snugly inserted in a respective one of grooves 19. The head portion 3 has an axially extending communication hole 10 which is formed forwardly of the slit 6 and spaced therefrom. At its forward end, the communication hole 10 is in communication with a plurality of small grooves 8 which extend sidewards and, in front of the small grooves 8, bristles 7 are studded in the bottom surface of a remote end part of the head portion. Formed in the shank part of the head portion 3 is an insertion hole 18 into which the conductor bar 2 can be slid, which insertion hole axially extends forwardly from the rearward end of the head portion 3, the conductor bar 2 terminating at a location within the hole 18 which is spaced from the bristles, at least part of the length of the conductor bar 2 being covered by the part of the head portion 3 that defines the hole 18.

When brushing teeth with the tooth-brush of the above construction, a toothpaste containing fluoride is used. During brushing, the bristles 7 of the head portion 3 are wetted with saliva which in turn runs up along the bristles 7 and reaches, via the small grooves 8, the communication hole 10 so that the conductor bar 2 is wetted with the saliva. In this manner, the bristles 7 are electrically connected to the conductor bar 2 by the saliva and as a result connected to a negative pole of the battery 15.

Since the fingers of the user grasping the grip portion 1 are electrically connected to the positive pole of the battery 15 via the cover plate 14 under which, in this embodiment, the battery 15 is disposed, a closed circuit is established through the tooth-brush and the user's body. Consequently, current from the battery 15 is passed to surfaces of the teeth via the fingers and the dental pulp tissue, and fluorine ions or negative ions conversely permeate from the surfaces of teeth into the teeth and precipitate therein to thereby strengthen quality of teeth and prevent tooth decay and pyorrhea. In use, the collar 13 blocks undesired saliva which would otherwise contaminate the fingers.

As the battery 15, a silver oxide battery is employed having, for example, a diameter of 5.8 mm, a thickness of 1.65 mm, a voltage of 1.5 volts and a current capacity of 8 mAH to provide a current of 20 to 30 µA which normally flows through the human body. When the body is wetted, a current of about 50 µA can be obtained. Thus, on the assumption that one tooth brushing is carried out for four minutes by holding the tooth-brush with wetted fingers, the aforementioned battery permits 2400 frequencies of tooth brushing. Accordingly, if tooth brushing is carried out twice a day, then the battery will serve for more than three years. Theoretically, the grip portion 1 can therefore serve by itself for the same period of time. On the contrary, the bristles 7 of the head portion 3 will be worn after being used for about one month and will become of no use.

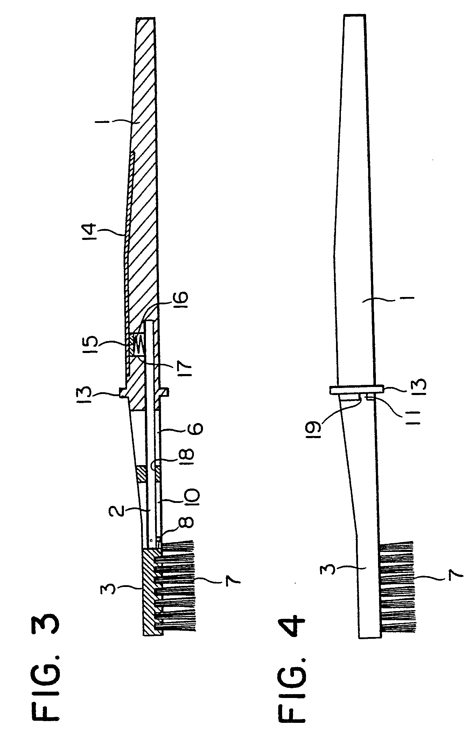

In the previous embodiment, upon occurrence of wear of the bristles, the grip portion 1 is retained for use and only the head portion 3 is replaced with a new one. In the handle assembled as shown in Figs. 2 to 4, when the grip portion 1 and the head portion 3 are pulled in opposite directions, the fork of arms 4 is opened to allow the boss 5 to be pulled out of the recess 9 and the conductor bar 2 to be also pulled out of the insertion hole 18, with the result that the head portion 3 is separated from the grip portion 1 as shown in Fig. 1. To put together the two portions 1 and 3, the reverse operation is effected. When these portions 1 and 3 are coupled together, the projections 11 are fitted in the grooves 19 to ensure that one portion can be prevented from rotating with respect to the other portion. The conductor bar 2 is also effective to prevent the head portion 3 in use from deforming relative to the grip portion 1 in the vertical direction in Fig. 3. As will be seen from the foregoing, the embodiment provides an economical tooth-brush wherein one grip portion 1 can be retained several times for use with several head portions 3.

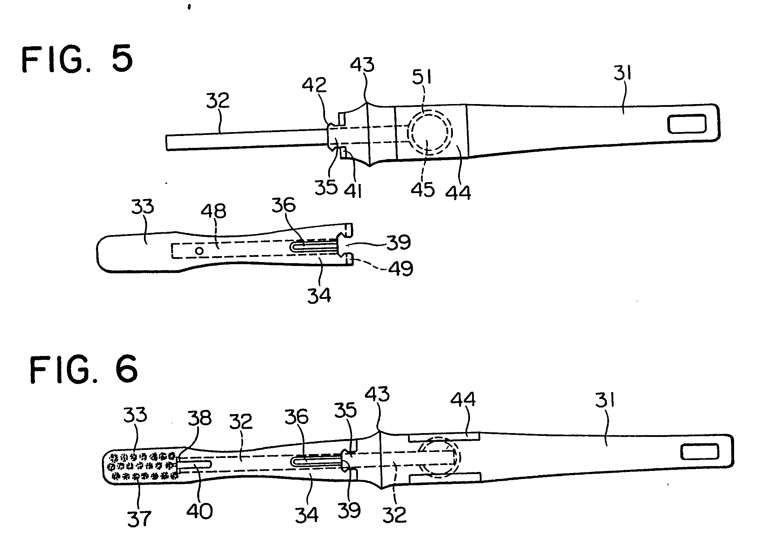

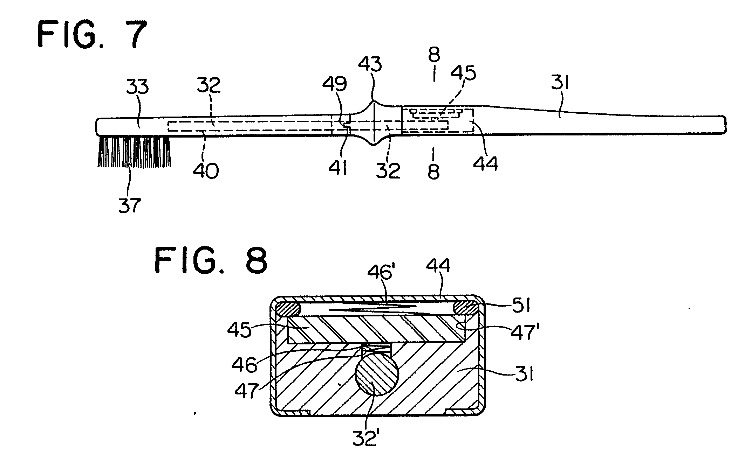

Referring to Figs. 5 to 8, a second embodiment of the invention will be described. In these figures, reference numerals designating like members in Figs. 1 to 4 illustrative of the first embodiment are added with thirty to designate like members in Figs. 5 to 8.

In the second embodiment, a grip portion 31 is curved backwardly from a peak of a water blocking collar 43 to form a curved outer surface for being grasped by fingers. A cover plate 44 is fitted in a partial annular groove formed in the outer periphery of the grip portion 31 rearwardly of the periphery of the collar 43 and it automatically rests on the grip portion by its resiliency. A battery 45 is received in a large diameter hole 47'. The battery 45 is electrically connected to a conductor bar 32 by a spring 46 received in a small diameter hole 47 and to the cover plate 44 by a spring 46'. The battery 45 is held in place by means of a resilient seal ring 51 interposed between the upper peripheral edge of the battery 45 and the cover plate 44.

Since in this embodiment the cover plate 44 can be supported on the grip portion 31 without resort to any particular, independent fixtures but by its own resiliency, the tooth-brush can be simplified in construction and is easy to manufacture. In addition, the resilient seal ring 51 steadily prevents intrusive water between the grip portion 31 and the cover plate 44 from reaching the battery 45.

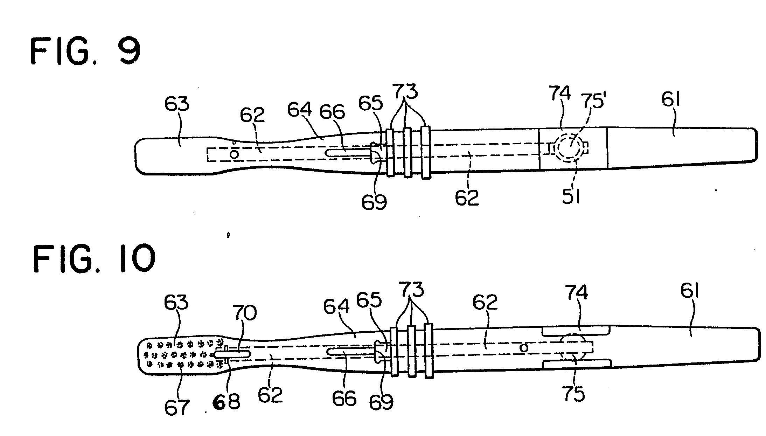

A third embodiment of the invention will now be described with reference to Figs. 9 to 12 in a similar manner to the second embodiment by adding reference numerals designating like members of the first embodiment with sixty to designate like members of the third embodiment.

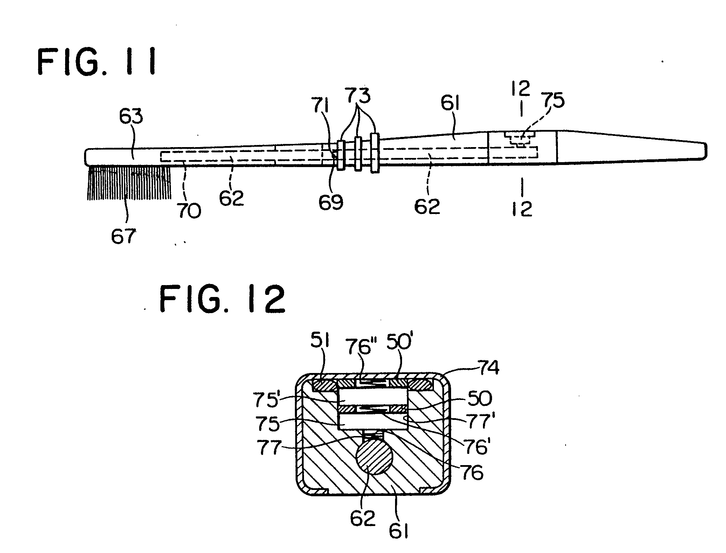

In the third embodiment, a grip portion 61 has a plurality of water blocking collars 73 whose heights are sequentially increased in proportion to distance from a head portion 63. As best seen from Fig. 12, a plurality of (two in this embodiment) batteries 75 and 75' are employed, and a spring 76 is interposed between a conductor bar 62 and the battery 75, a spring 76' between the batteries 75 and 75' and a spring 76'' between the battery 75' and a cover plate 74. The spring 76 is received in a small diameter hole 77, and the batteries 75 and 75' are received in a large diameter hole 77'. The springs 76' and 76'' are surrounded by annular insulating members 50 and 50', respectively. The annular insulating member 50' is also surrounded by a resilient seal ring 51 interposed between an upper surface of the grip portion 61 and a rear surface of the cover plate 74. This embodiment is different from the previous two embodiments in that projections 71 are provided for the grip portion 61 and co-operating grooves are provided for a head portion 63.

If in the third embodiment each of the plurality of batteries 75 and 75' has a capacity which is equal to the capacity of the battery 15 in the first embodiment, the total voltage becomes an integral number times (twice in this embodiment) the voltage of the battery 15. If the total voltage is desired to be equal to the voltage of the battery 15, the capacity of each of the batteries 75 and 75' needs only be a fraction of the capacity of the battery 15. In the third embodiment, however, the batteries 75 and 75' are connected in series and are therefore designed differently from the battery 15 of the first embodiment. The batteries 75 and 75' are insulated from each other by the insulating member 50 interposed therebetween, and the battery 75' is insulated from the cover plate 74 by the insulating member 50' interposed therebetween.

Additionally, the plurality of collars 73 in this embodiment enhance prevention of movement of saliva from the head portion 63 to the grip portion 61. Moreover, like the second embodiment, the resilient seal ring 51 fulfils a sealing effect that prevents intrusive water between the grip portion 61 and the cover plate 74 from reaching the batteries 75' and 75.

In the foregoing embodiments, the grip and head portions are typically moulded from an electrically non-conductive synthetic resin and toothpaste containing fluoride is used with the tooth-brush. However, other kinds of toothpaste were used practically to confirm that a similar tendency to that by the toothpaste containing fluoride could be obtained.

## Claims

1. An electric tooth-brush comprising a grip portion (1) which can be gripped manually, an elongate head portion (3) connected to and separable from said grip portion and studded with bristles (7), a head conductor member (2), a grip conductor member (14) which covers a surface of said grip portion and an electric power source (15) connected to said head conductor member and said grip conductor member, characterised in that:-

said head conductor member is an elongate head conductor member which projects lengthwise from the end of said grip portion adjacent said head portion, said head portion has an elongate lengthwise extending receiving space (18) in it, said head conductor member being carried by said grip portion and extending slidably into said receiving space and terminating at a location within said receiving space, which location is spaced from said bristles, and at least a part of the length of said head conductor member being covered by the part of the head portion that defines said receiving space; and

fluid communication passage means (8, 10) extends from said bristles to said receiving space so that fluid can flow from said bristles to said head conductor member in said receiving space.

2. An electric tooth-brush according to Claim 1, characterised in that said fluid communication passage means (8, 10) comprises a surface of said head portion (3) which is studded with said bristles (7) and a passage (10) extending from said surface of said head portion to said receiving space (18), whereby fluid can flow from said bristles to said receiving space via said surface of said head portion.

3. An electric tooth-brush according to Claim 2, characterised in that said fluid communication passage (8, 10) means further comprises at least one groove (8) provided on said surface of said head portion (3) and extending substantially from said bristles (7) to said passage (10) for guiding fluid to flow from said bristles to said passage.

4. An electric tooth-brush according to any preceding claim, characterised in that said head conductor member (2) is a bar and said receiving space (18) is a hole which opens through the end of said head portion (3) adjacent said grip portion (1) to receive said bar.

5. An electric tooth-brush according to Claim 4 as dependent on Claim 2 or 3, characterised in that said passage (10) comprises an opening from said surface of said head portion which is in communication with said hole (18).

6. An electric tooth-brush according to any preceding claim, characterised in that said head portion (3) comprises an electrically non-conductive synthetic resin material.

7. An electric tooth-brush according to any preceding claim, characterised in that said head portion (3) comprises an elongate shank part extending away from said grip portion (1) and a remote end part on which said bristles (7) are studded, said receiving space (18) extending lengthwise through said shank part.

## Patentansprüche

1. Elektrische Zahnbürste, umfassend einen mit der Hand ergreifbaren Griffbereich (1), einén länglichen Kopfbereich (3), der mit dem Griffbereich lösbar verbunden und mit Borsten (7) versehen ist, ein Kopfleiterteil (2) und ein Griffleiterteil (14), das eine Fläche des Griffbereichs und eine mit dem Kopfleiterteil und dem Griffleiterteil verbundene elektrische Spannungsquelle (15) bedeckt, dadurch gekennzeichnet,

daß das Kopfleiterteil ein längliches Kopfleiterteil ist, das von dem dem Kopfbereich benachbarten Ende des Griffbereichs in Längsrichtung vorsteht, wobei der Kopfbereich einen sich in Längsrichtung erstreckenden länglichen Aufnahmehohlraum (18) aufweist, wobei das Kopfleiterteil von dem Griffbereich getragen ist und sich verschiebbar in den Aufnahmehohlraum hineinerstreckt und an einer Stelle in diesem Aufnahmehohlraum endet, wobei sich diese Stelle im Abstand von den Borsten befindet, und wobei mindestens ein Längenbereich dieses Kopfleiterteils von demjenigem Teil des Kopfbereichs bedeckt ist, der diesen Aufnahmehohlraum begrenzt, und

daß sich Flüssigkeits-Verbindungsmittel (8, 10) von den Borsten zu dem Aufnahmehohlraum erstrecken, so daß Flüssigkeit von den Borsten zu dem Kopfleiterteil in dem Aufnahmehohlraum fließen kann.

2. Elektrische Zahnbürste nach Anspruch 1, dadurch gekennzeichnet, daß die Flüssigkeits-Verbindungsmittel (8, 9) eine mit Borsten (7) versehene Fläche des Kopfbereichs (3) und einen Kanal (10) umfassen, der sich von dieser Fläche des Kopfbereichs zu dem Aufnahmehohlraum (18) erstreckt, wodurch Flüssigkeit von den Borsten über diese Fläche des Kopfbereichs zu dem Aufnahmehohlraum fließen kann.

3. Elektrische Zahnbürste nach Anspruch 2, dadurch gekennzeichnet, daß die Flüssigkeits-Verbindungsmittel (8, 10) ferner mindestens eine Nut (8) umfassen, die auf der Oberfläche des Kopfbereichs (3) ausgebildet ist und sich im wesentlichen von den Borsten (7) zu dem Kanal (10) erstreckt, um Flüssigkeit von den Borsten zu diesem Kanal zu leiten.

4. Elektrische Zahnbürste nach einem der vorhergehenden Ansprüche, dadurch gekennzeichnet, daß das Kopfleiterteil (2) eine Stange ist und daß der Aufnahmehohlraum (18) eine Bohrung ist, die an dem dem Griffbereich (1) benachbarten Ende des Kopfbereichs (3) austritt, um diese Stange aufzunehmen.

5. Elektrische Zahnbürste nach Anspruch 4 in Verbindung mit Anspruch 2 oder 3, dadurch gekennzeichnet, daß der Kanal (10) eine mit der Bohrung (18) in Verbindung stehende Öffnung in der Oberfläche des Kopfbereiches umfaßt.

6. Elektrische Zahnbürste nach einem der vorhergehenden Ansprüche, dadurch gekennzeichnet, daß der Kopfbereich (3) ein elektrisch nichtleitfähiges Kunstharzmaterial umfaßt.

7. Elektrische Zahnbürste nach einem der vorhergehenden Ansprüche, dadurch gekennzeichnet, daß der Kopfbereich (3) ein längliches Schaftteil umfaßt, das sich von dem Griffbereich (1) weg erstreckt, und ein abliegendes Endteil umfaßt, auf dem die Borsten (7) angeordnet sind, wobei sich der Aufnahmehohlraum (18) in Längsrichtung durch das Schaftteil erstreckt.

**Revendications**

1. Brosse à dents électrique comprenant une poignée (1) qu'on peut saisir à la main, une tête allongée (3) rendue solidaire de cette poignée, en pouvant en être séparée, et garnie de soies (7), un élément conducteur de tête (2), un élément conducteur de poignée (14) qui recouvre une surface donnée de la poignée et une source de courant électrique (15) reliée à cet élément conducteur de tête et cet élément conducteur de poignée, caractérisée en ce que l'élément conducteur de tête est un élément conducteur de tête allongé qui fait saillie dans le sens de la longueur à partir de l'extrémité de la poignée voisine de la tête, et cette tête comporte, en son intérieur, un espace récepteur (18) allongé et s'étendant dans le sens de la longueur, l'élément conducteur de tête étant porté par la poignée et s'étendant de manière coulissante dans cet espace récepteur, en se terminant en un emplacement situé à l'intérieur de cet espace récepteur, cet emplacement étant à une certaine distance des soies, et au moins une partie de la longueur de cet élément conducteur de tête étant recouverte par la partie de la tête qui délimite l'espace récepteur, et en ce que des moyens formant passage de communication de fluide (8, 10) s'étendent des soies à cet espace récepteur de façon qu'un fluide puisse s'écouler de ces soies à l'élément conducteur de tête situé dans l'espace récepteur.

2. Brosse à dents électrique suivant la revendication 1, caractérisée en ce que les moyens formant passage de communication de fluide (8, 10) comprennent une surface de la tête (3) qui est garnie des soies (7) et un passage (10) s'étendant de la surface de la tête jusqu'à l'espace récepteur (18), de sorte qu'un fluide peut s'écouler des soies à cet espace récepteur par l'intermédiaire de cette surface de la tête.

3. Brosse à dents électrique suivant la revendication 2, caractérisée en ce que les moyens formant passage de communication de fluide (8, 10) comprennent en outre au moins une rainure (8) ménagée sur ladite surface de la tête (3) et s'étendant sensiblement des soies (7) au passage (10) afin de guider un fluide pour qu'il s'écoule des soies à ce passage.

4. Brosse à dents électrique suivant l'une quelconque des revendications précédentes, caractérisée en ce que l'élément conducteur de tête (2) est une barre et l'espace récepteur (18) est un trou qui débouche sur l'extrémité de la tête (3) en une position adjacente à la poignée (1) afin de pouvoir recevoir cette barre.

5. Brosse à dents électrique suivant la revendication 4 lorsqu'elle dépend de la revendication 2 ou 3, caractérisée en ce que le passage (10) est constitué par une ouverture ménagée à partir de ladite surface de la tête et qui débouche dans le trou (18).

6. Brosse à dents électrique suivant l'une quelconque des revendications précédentes, caractérisée en ce que la tête (3) est constituée d'une matière formée d'une résine synthétique électriquement non conductrice.

7. Brosse à dents électrique suivant l'une quelconque des revendications précédentes, caractérisée en ce que la tête (3) comprend une partie allongée en forme de tige s'éloignant de la poignée (1) et une partie extrême, la plus éloignée, qui est garnie des soies (7), l'espace récepteur (18) s'étendant dans le sens de la longueur à travers cette tige.

FIG. I

FIG. 2

FIG. 3

FIG. 4

# FIG. 5

# FIG. 6

EP 0 174 740 B1

# FIG. 7

# FIG. 8

FIG. 9

FIG. 10

FIG. 11

63    71  73    61    12  75

67  70  62    69    62    12

FIG. 12

51  76"  50'

75'    74

75    50

77    77'

77    76'

76

62  61

EP 0 174 740 B1